# EUROPEAN PATENT APPLICATION

(11) **EP 1 598 344 A1**
(43) Date of publication of application: **23.11.2005**
(21) Application number: 04705912.6
(22) Date of filing: 28.01.2004
(51) Int. Cl.: C07D 265/34, H05B 33/14

(54) **NILE RED LIGHT-EMITTING COMPOUND, METHOD FOR PRODUCING NILE RED LIGHT-EMITTING COMPOUND, AND LIGHT-EMITTING DEVICE**

(30) Priority: 31.01.2003 JP 2003025188
(71) Applicant: HIROSE ENGINEERING CO., LTD., Tokyo 146-0092 (JP); Kanto Denka Kogyo CO., LTD., Chiyoda-ku, Tokyo 100-0005 (JP)
(72) Inventor: NAKAYA, Tadao c/p Hirose Engineering Co., Ltd., Tokyo 146-0092 (JP); TAJIMA, Akio c/o Hirose Engineering Co., Ltd., Tokyo 146-0092 (JP); TOBITA, Michaiki c/o Hirose Engineering Co., Ltd., Tokyo 146-0092 (JP); SAIKAWA, Tomoyuki c/o Hirose Engineering Co., Ltd., Tokyo 146-0092 (JP); GAO, Yuan, c/o Kanto Denka Kogyo Co., Ltd, Shibukawa-shi, Gunma 377-0027 (JP); OKUBO, Kimitaka, c/o Kanto Denka Kogyo Co., Ltd, Shibukawa-shi, Gunma 377-0027 (JP)
(74) Representative: Olgemöller, Luitgard Maria
(86) International application number: PCT/JP2004/000771
(87) International publication number: WO 2004/067519

(57) **Abstract**

The objectives of the present invention are to provide a red light-emitting compound capable of emitting red light at high color purity and strong luminance, and excellent in fastness, and to provide a luminescent element capable of emitting red light at strong luminance. The objectives are achieved by the Nile Red luminescent compound emitting red light represented by formula (1): 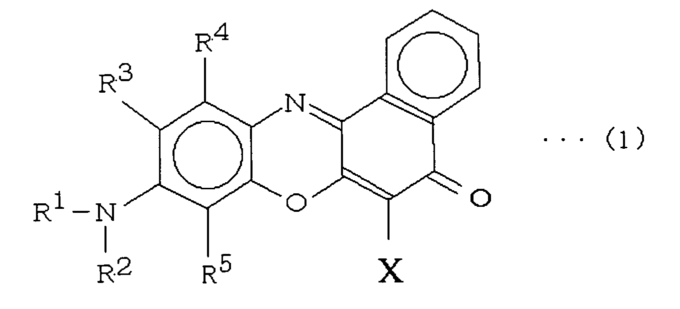 wherein X is cyano group or a fluorohydrocarbyl group, and a luminescent element, the light-emitting layer of which includes the compound.

## Description

### Technical Field

The present invention relates to a Nile red luminescent compound emitting red light, a process for producing the same and a luminescent element utilizing the same. More particularly, this invention relates to a Nile red luminescent compound capable of emitting a light the color of which is nearly crimson, at a high luminance upon the application of electric energy, a novel process of producing the compound and a luminescent element utilizing the same.

### Background Art

For organic electroluminescent elements, which are often abbreviated to "organic EL elements", have been proposed various organic compounds.

However, compounds that are capable of emitting red light at a high luminance and endurable against heat, light, etc. have not been developed.

The objective of this invention is to provide an organic compound capable of emitting red light at a high luminance, and/or capable of emitting a light the color of which is such a red that the value on the x-axis in the CIE chromaticity is over 0.63, and further endurable against heat, light, etc. This invention also aims for providing a process for producing the organic compound and a luminescent element utilizing the compound.

### Summary of the Invention

In order to solve the aforementioned problems, this invention provides a Nile red luminescent compound emitting red light that has a structure represented by formula (1): 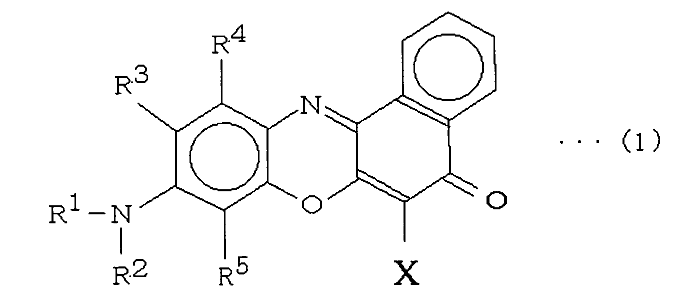 wherein R¹ is a lower alkyl group having from 1 to 5 carbon atoms or benzyl group, or forms -CH₂CH₂-CR⁶R⁷- together with R³ (wherein the carbon atom of the -CR⁶R⁷- part is bound to the benzene fragment of chemical formula (1), each of R⁶ and R⁷ is a hydrogen atom, a lower alkyl group having from 1 to 5 carbon atoms, or benzyl group, and R⁶ and R⁷ may be the same or different from each other).

R² is a lower alkyl group having from 1 to 5 carbon atoms or benzyl group, or forms -CH₂CH₂R⁸R⁹- together with R⁵ (wherein the carbon atomof the -CR⁸R⁹- part is bound to the benzene fragment of chemical formula (1), each of R⁸ and R⁹ is a hydrogen atom, a lower alkyl group having from 1 to 5 carbon atoms, or benzyl group, and R⁸ and R⁹ may be the same or different from each other).

R³ is a hydrogen atom, forms -CH₂CH₂-CR⁶R⁷- with R¹, or forms with R⁴ a naphthalene ring including as a part thereof the benzene fragment of chemical formula (1).

R⁹ is a hydrogen atom, or forms with R³ a naphthalene ring including as a part thereof the benzene fragment of chemical formula (1).

R⁵ is a hydrogen atom, or forms -CH₂CH₂-CR⁸R⁹- with R².

X is cyano group or a fluorohydrocarbyl group.

Another solution to the above-mentioned problems is a process for producing the Nile red luminescent compound emitting red light represented by formula (1), comprising reacting a Nile red pigment compound represented by formula (2) with a halogenating agent to produce a halogenated Nile red intermediate represented by formula (3), and replacing the halogen atom with a fluorohydrocarbyl group or cyano group. 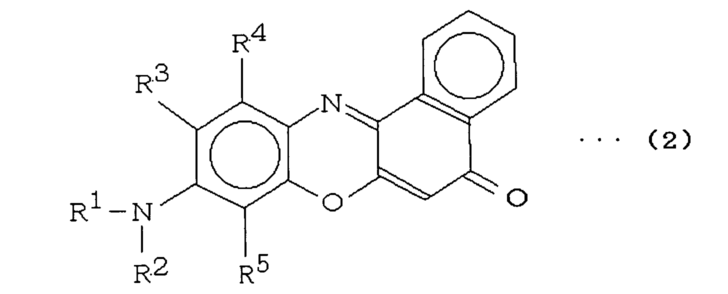 In the formula, R¹, R², R³, R⁴ and R⁵ mean the same atoms and groups as those defined above. 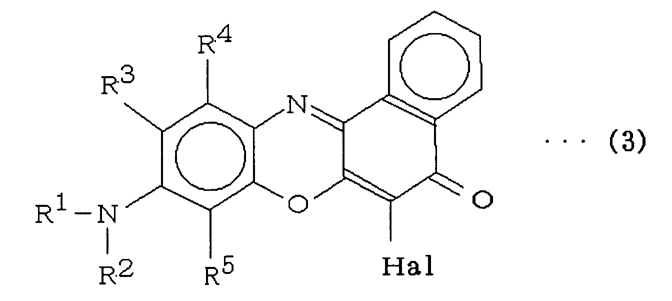 In the formula, R¹, R², R³, R⁴ and R⁵ mean the same atoms and groups as those defined above, and "Hal" denotes a halogen atom.

Still another solution to the above-mentioned problem is a luminescent element comprising a pair of electrodes and a light-emitting layer including the Nile red luminescent compound represented by formula (1) between the electrodes.

### Brief Description of the Drawings

Figure 1 is an illustration showing an example of the luminescent element according to the present invention.
Figure 2 is an illustration showing another example of the luminescent element according to the present invention.
Figure 3 is an illustration showing a still another example of the luminescent element according to the present invention.
Figure 4 is an illustration showing a further example of the luminescent element according to the present invention.
Figure 5 is an IR chart of the bromo-Nile red intermediate synthesized in Example 1.
Figure 6 is an NMR chart of the bromo-Nile red intermediate synthesized in Example 1.
Figure 7 is a fluorescence spectrumof the trifluoromethyl group-introduced Nile red luminescent compound synthesized in Example 1.
Figure 8 is an NMR chart of the Nile red luminescent compound synthesized in Example 1.
Figure 9 is an IR chart of the Nile red luminescent compound synthesized in Example 1.
Figure 10 is a fluorescence spectrum of the cyano group-introduced Nile red luminescent compound synthesized in Example 2.
Figure 11 is an NMR chart of Nile red luminescent compound synthesized in Example 2.
Figure 12 is an IR chart of Nile red luminescent compound synthesized in Example 2.

### Detailed Description of the Preferred Embodiments

The Nile red luminescent compound according to the present invention is represented by formula (1):

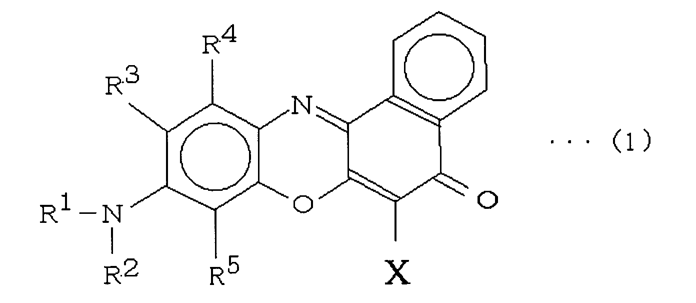

In this formula, R¹ is a lower alkyl group having 1-5 carbon atoms, or benzyl group. The lower alkyl group includes methyl group, ethyl group, a propyl group, a butyl group and a pentyl group.

R² is a hydrogen atom or a lower alkyl group having 1-5 carbon atoms, or benzyl group. The lower alkyl group includes the same groups as R¹. R¹ and R² may be the same lower alkyl group or different from each other.

Together with R³, R¹ forms -CH₂CH₂-CR⁶R⁷- (wherein the carbon atom of the -CR⁶R⁷- part is bound to the benzene fragment of chemical formula (1), each of R⁶ and R⁷ is a hydrogen atom, a lower alkyl group having 1-5 carbon atoms, or benzyl group, and R⁶ and R⁷ may be the same or different from each other).

When R¹ and R² are lower alkyl groups, preferable -NR¹R² includes diethylamino group, di-n-propylamino group, di-i-propylamino group, a butyl group, etc.

Together with R⁵, R² forms -CH₂CH₂-CR⁸R⁹- (wherein the carbon atom of the -CR⁸R⁹- part is bound to the benzene fragment of chemical formula (1), each of R⁸ and R⁹ is a hydrogen atom, a lower alkyl group having 1-5 carbon atoms, or benzyl group, and R⁸ and R⁹ may be the same or different from each other).

When R¹ forms -CH₂CH₂-CR⁶R⁷- with R³, and R² forms -CH₂CH₂-CR⁸R⁹- with R⁵, formula (1) becomes the following formula (4) :

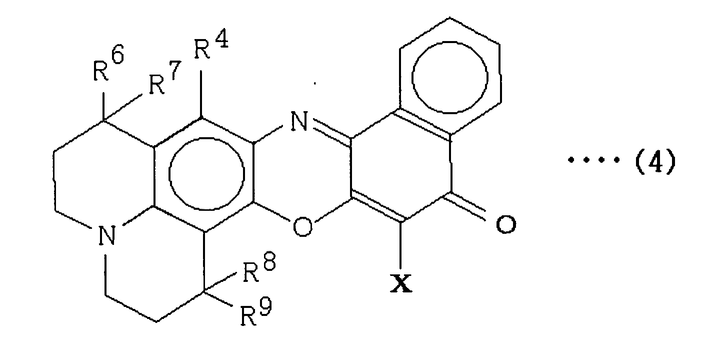

In this formula (4), R⁴, R⁶, R⁷, R⁸, R⁹ and X denote the same as those mentioned above.

Both R³ and R⁴ may be hydrogen atoms, or form together a naphthalene ring including as a part thereof the benzene fragment of chemical formula (1). The red light-emitting luminescent compound that has the naphthalene ring including as a part thereof the benzene fragment formed by R³ and R⁴ is represented by formula (5).

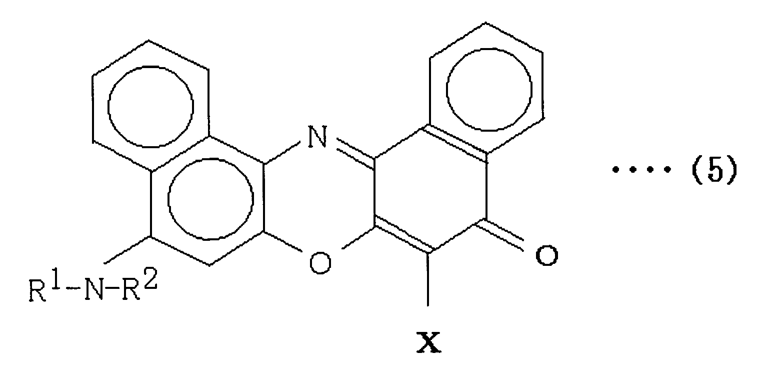

In formula (5), R¹, R² and X denote the same as those mentioned above.

In formula (1), X may be a fluorohydrocarbyl group or cyano group.

The fluorohydrocarbyl group includes groups made by replacing one or more hydrogen atoms thereof with fluorine atoms. Specifically, it includes fluoro-lower-hydrocabyl groups made by replacing one or more hydrogen atoms of saturated or unsaturated hydrocarbyl groups having from 1 to 10 carbon atoms with fluorine atoms. In particular, suitable fluorohydrocarbyl groups are fluoro-lower-saturated-hydrocarbyl groups made by replacing one or more hydrogen atoms of saturated hydrocarbyl groups that have from 1 to 10, preferably from 1 to 5, carbon atoms with fluorine atoms. More preferable are perfluorohydrocarbyl groups made by replacing all the hydrogen atoms of saturated hydrocarbyl groups that have from 1 to 5 carbon atoms with fluorine atoms. Specific examples of the fluoro-lower-saturated-hydrocarbyl group are -CH₂F, -CHF₂, -CF₃, -CH₂CF₃, -CHFCF₃, -CF₂CF₃, -CH₂CH₂CF₃, -CH₂CHFCF₃, -CH₂CF₂CF₃, -CH₂FCF₂CF₃, etc. Examples of the perfluoroalkyl group are -CF₃, -CF₂CF₃, -CF₂(CF₂)ₙCF₃, wherein n denotes an integer from 1 to 3. The most preferable are -CF₃ and -CF₂CF₃.

In the Nile red luminescent compound represented by formula (1), -NR¹R² is an electron-donating group and the fluorohydrocarbyl group or cyano group represented by X is an electron attractive group, so that π electron cloud on the skeleton of the Nile red compound is extended to the substituents. Therefore, we surmise that the application of a little energy enables the luminescent compound to emit red light. The novel luminescent compound of this invention is characterized by the structure where R¹-N-R², the electron-donating group, provides the π electron cloud with electrons. Because this Nile red skeleton has an electronically stable structure and therefore the Nile red luminescent compound is chemically stable, the luminescent compound does not deteriorate even under severe environments, which is a special characteristic of the compound.

The Nile red luminescent compound emitting red light represented by formula (1) may be prepared by the following method.

The compound may be obtained by reacting a Nile red compound represented by general formula (2) with a halogenating agent.

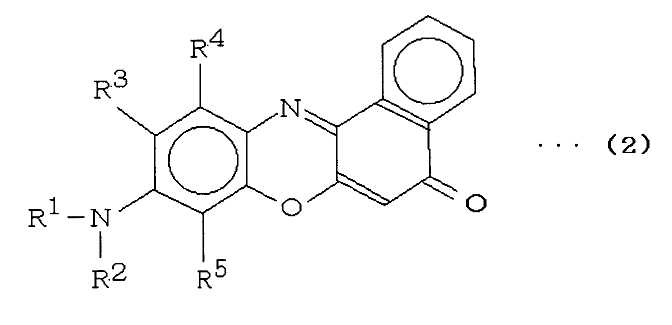

The halogenating agent may be a common one that is able to replace hydrogen atoms on an aromatic ring with halogen atoms. Specific examples of the halogenating agent are sulfuryl chloride, phosphorus pentachloride, etc. when hydrogen atoms on an aromatic ring are replaced with chlorine atoms. Generally, when hydrogen atoms on an aromatic ring are replaced with halogen atoms, an imido-N-halosuccinate such as an imido-N-bromosuccinate, and a dialkyl halomalonate such as a dialkyl bromomalonate may be used.

The Nile red compound represented by formula (2) and the halogenating agent react easily by heating them in a solvent. The solvent includes acetic anhydride, acetic acid, an acid anhydride having not more than 5 carbon atoms, an aromatic solvent such as benzene or toluene, a chlorinated solvent such as dichloromethane or chloroform, a dioxane, etc. The reaction temperature usually ranges between 0 and 250°C, preferably between 20 and 170°C. After the reaction, purification and separation by an ordinary method will provide the targeted halogenated Nile red intermediate represented by formula (3). 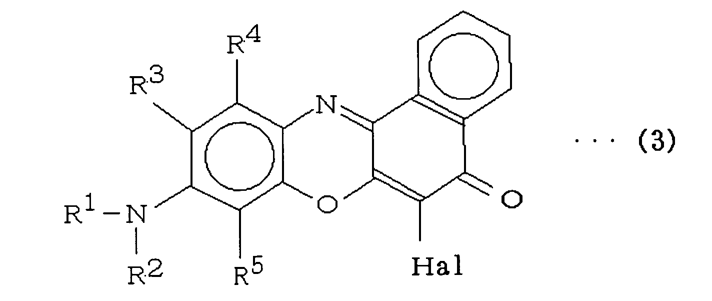 In formula (3), R¹, R², R³, R⁴ and R⁵ mean the same atoms and groups as those defined above, and "Hal" denotes a halogen atom.

The halogenated Nile red intermediate is converted to the Nile red luminescent compound represented by formula (1) by replacing the halogen atom thereof with the halohydrocarbyl group or cyano group.

To introduce the halohydrocarbyl group into the intermediate, a method of reacting the intermediate with a metal perfluorohydrocarbyl reagent, which is generated in the reaction system, such as a copper perfluoroalkyl reagent, a method of generating a perfluoroalkyl radical, which is followed by the reaction with the halogenated Nile red intermediate, a method of dehydration by adding a metal perfluoroalkyl reagent, such as a Grignard reagent, a lithium reagent, or an aluminum reagent, to a carbonyl compound, or other methods may be employed. Although the halogen atom "Hal" of the intermediate may be any one of iodine, bromine, fluorine, and chlorine, the employment of iodine or bromine is recommended because iodine and bromine are easy to handle and the employment thereof produces the targeted with high yields.

Among the above-mentioned methods, the method of producing a copper perfluoroalkyl reagent in the reaction system from a perfluoroalkyl iodide, which is represented by the formula CₘF₂ₘ₊₁I wherein m is an integer from 1 to 20, preferably from 1 to 10, more preferably from 1 to 5, and copper powder, which is followed by the reaction of this copper perfluoro alkyl reagent with the halogenated Nile red intermediate represented by formula (3) is preferable. The raw materials for this method are not expensive, and easy to obtain and handle. Moreover, the yield of the reaction product is high.

The introduction of a cyano group into the halogenated Nile red intermediate represented by formula (3) is suitably carried out by reacting the intermediate with a transition metal cyanide. The cyanidation reaction is usually done in a polar aprotic solvent, examples of which are aromatic amines such as pyridine or quinoline, dimethylformamide (DMF), N-methylpyrrolidone, hexamethylphosphoric triamide (HMPA), etc.

The halogenated Nile red intermediate can be easily produced only by heating a mixture of the Nile red compound and the halogenating agent. Furthermore, the introduction of a cyano group or a fluorohydrocarbyl group into the intermediate proceeds quickly by heating. Therefore, this simple production method of the Nile red luminescent compound emitting red light is an industrial method.

The luminescent element according to the present invention will be explained hereinafter.

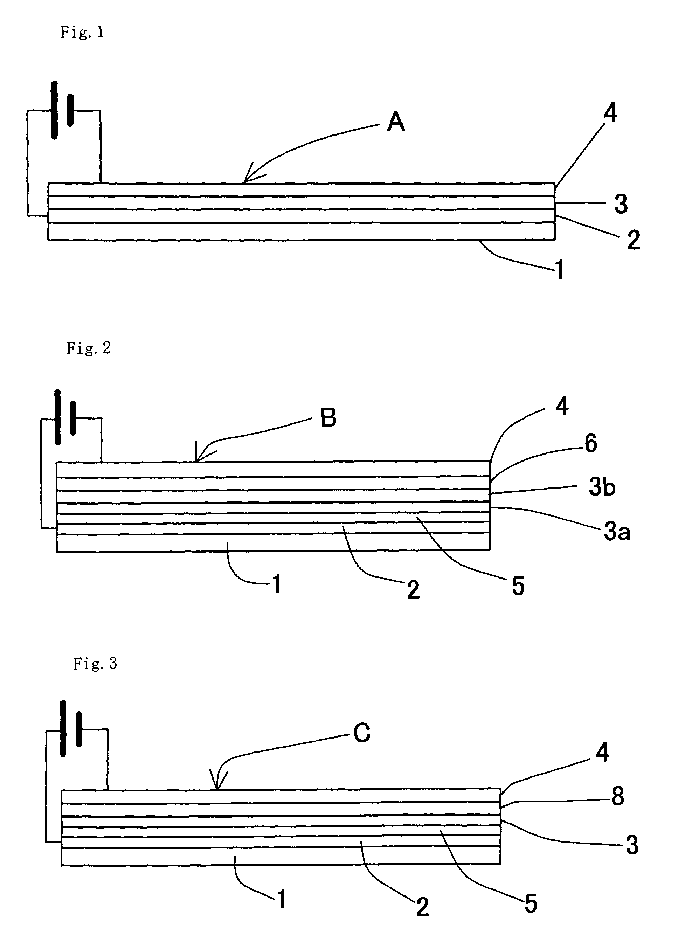

Figure 1 is a schematic illustration that shows the sectional structure of a luminescent element, which is a single-layer organic EL element, according to the present invention. As shown in this figure, the luminescent element A is prepared by layering a light-emitting layer 3 and an electrode layer 4 in this order on a substrate 1 with which a transparent electrode 2 has been provided.

When the luminescent element shown in Figure 1 includes the Nile red luminescent compound emitting red light of the present invention, a blue light-emitting compound and a green light-emitting compound at a balanced composition, it emits white light upon the application of electricity through the transparent electrode 2 and the electrode layer 4. The total amount of the Nile red luminescent compound of the present invention, the blue light-emitting compound and the green light-emitting compound, and the proportion of the amount of the Nile red luminescent compound to that of the blue light-emitting compound to that of the green light-emitting compound, included in the layer 3 to let the element emit white light, vary depending on the kind of each compound. They are decided for each luminescent element depending on the kind of each compound included therein. When the luminescent element is intended to emit red light, the light-emitting layer 3 may include only the Nile red luminescent compound of the present invention. Also, when this luminescent element is intended to emit light of any color other than white and red, the total amount of the compounds and their respective amounts should be changed depending on the color. For example, when the luminescent element of this invention is intended to emit white light, the ratio of the amount of the Nile red luminescent compound to that of the blue light-emitting compound to that of the green light-emitting compound is usually 5-200:10-100:50-20000 in weight, preferably 10-100:50-500: 100-10000.

Examples of the blue light-emitting compound are diphenylvinyl biphenol compounds emitting blue light and stilbene compounds emitting blue light. A preferable diphenylvinyl biphenol compound emitting blue light is DPVBi represented by formula (10).

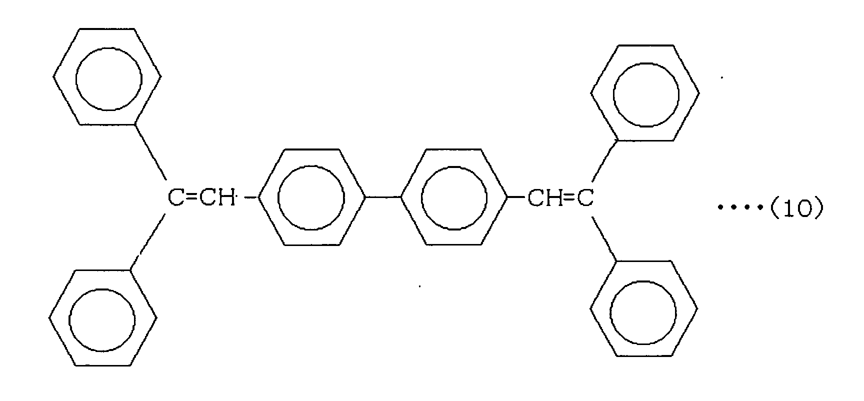

For the green light-emitting compound is suitable a coumarin compound emitting green light, an indophenol compound emitting green light, or an indigo compound emitting green light. The coumarin compound represented by formula (11) is preferable among them.

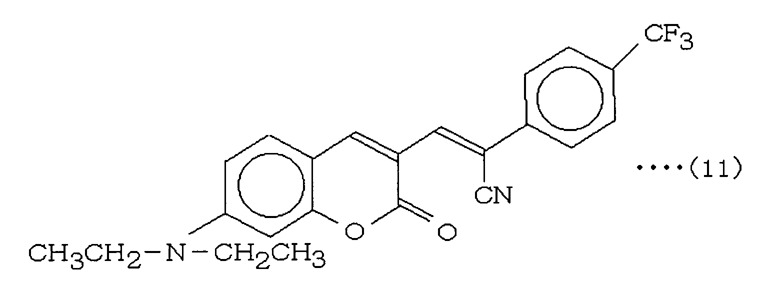

Upon the application of an electric field between the transparent electrode 2 and the electrode layer 4, electrons are injected from the electrode layer 4 and positive holes are injected from the transparent electrode 2. In the light-emitting layer 3, the electrons are recombined with positive holes, which causes the energy level to return to the valence band from the conduction band. This transition of the energy level is accompanied by emission of the energy differential as light.

The luminescent element A shown in Figure 1, when it is shaped to a planar one with a large area, may be used as a planar illuminator, for example a large-area wallilluminator emitting white light when fixed on a wall, or a large-area ceiling illuminator emitting white light when fixed on a ceiling. This light-emitting element may be used as a planar light source in place of a point light source, such as a conventional bulb, and a line light source, such as a conventional fluorescent lamp. In particular, this white light-emitting illuminator can suitably be used to light up walls, ceilings and floors in dwelling rooms, offices and passenger trains, or to make them emit light. Moreover, this luminescent element A may be suitable for the backlight used in displays of computers, cellular phones and ATMs. Furthermore, this illuminator may be used for various light sources, such as a light source of direct illumination and a light source of indirect illumination. Also, it may be used for the light sources of advertisement apparatuses, road traffic sign apparatuses and light-emitting billboards, which have to emit light at night and provide good visibility. It may also be used as a light source for brake lights of vehicles such as cars. In addition, because the light-emitting element A includes the Nile red luminescent compound of the present invention, which has the special chemical structure, in the light-emitting layer, it has a long life. Therefore, light sources employing the luminescent element A will naturally have a long life.

As understood from the foregoing, when the light-emitting layer of the luminescent element A includes the Nile red luminescent compound emitting red light of the present invention and not the blue light-emitting compound or the green light-emitting compound, the luminescent element A emits clear red light.

The luminescent element A may also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because this luminescent element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate, the surface of which is insulated, for example, by forming an insulating layer thereon.

When the substrate 1 is opaque, the luminescent element, which includes the blue light-emitting compound, the green light-emitting compound and the Nile red luminescent compound of the present invention, is a single-faced illuminator that emits white light from one side of the element. On the other hand, when the substrate 1 is transparent, the luminescent element is a double-faced illuminator that emits white light from both of the substrate 1 and the surface layer opposite to the substrate.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 includes the Nile red luminescent compound of the present invention when the layer 3 is intended to emit red light. It includes a blue light-emitting compound and a green light-emitting compound in addition to the Nile red luminescent compound of the present invention when it is intended to emit white light. The light-emitting layer 3 may be a high polymer film prepared by dispersing the Nile red luminescent compound emitting red light of the present invention, or a blue light-emitting compound, a green light-emitting compound and the Nile red luminescent compound of the present invention in a high polymer. Also, the light-emitting layer 3 may be a deposited film which is prepared by depositing the Nile red luminescent compound of the present invention, or a blue light-emitting compound, a green light-emitting compound and the Nile red luminescent compound of the present invention on the transparent electrode 2.

Examples of the high polymer for the high polymer film are a polyvinyl carbazole, a poly (3-alkylthiophen), a polyimide including an arylamide, a polyfluorene, a polyphenylene vinylene, poly-α-methylstyrene, and a copolymer of vinylcarbazole and α-methylstyrene. Among them, a polyvinyl carbazole is preferable.

The amount of the Nile red luminescent compound emitting red light, or that of a blue light-emitting compound, a green light-emitting compound and the Nile red luminescent compound in the high polymer film is, typically, 0.01 to 2 weight%, preferably, 0.05 to 0.5 weight%.

The thickness of the high polymer film ranges, typically, between 30 nm and 500 nm, preferably between 100 nm and 300 nm. When the thickness is too small, the amount of the emitted light may be insufficient. On the other hand, when the thickness is too large, voltage required to drive the element may be too high, which is not desirable. Besides, the large thickness may reduce the flexibility of the element necessary to shape a planar, tubular, curved or ring article.

A typical example of forming the high polymer film on the transparent electrode may be the application of a solution of the Nile red luminescent compound or the mixture of a blue light-emitting compound, a green light-emitting compound and the Nile red luminescent compound dissolved in a suitable solvent onto the transparent electrode. The application method includes, for example, a spin cast method, a coating method, a dip method, etc.

When the light-emitting layer 3 is made of a deposited film, the thickness of the film is typically 0.1 to 100 nm, although it varies depending on the structure of the light-emitting layer 3. When the thickness is too small or too large, the deposited film layer will have the same problems as the high polymer film layer described above.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode may easily be formed on the surface of light-emitting layer 3, which, in turn, has been formed on substrate 1, by the technique of metal deposition.

When either of the deposition or the application is employed for the formation of the light-emitting layer, a buffer layer should be inserted between the electrode layer and the light-emitting layer.

Materials for the buffer layer are, for example, an alkaline metal compound such as lithium fluoride, an alkaline earth metal compound such as magnesium fluoride, an oxide such as an aluminum oxide, and 4, 4'-biscarbazole biphenyl (Cz-TPD). Also, materials for forming a buffer layer between the cathode made of ITO, etc. and the organic layer are, for example, m-MTDATA (4,4',4"-tris (3-methylphenyl-phenylamino) triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescent element, improve the quantum efficiency of luminescence, and achieve an increase in the luminance of the emitted light.

Next, the second example of the luminescent element according to this invention is shown in Figure 2. This figure is an illustration showing the sectional layer structure of an example of the luminescent element, which is a multi-layer organic EL element.

As shown in Figure 2, the luminescent element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises the light-emitting sublayers 3a and 3b. The light-emitting sublayer 3a is a deposited film including light-emitting compounds. The light-emitting sublayer 3b is a DPVBi layer that functions as a host.

Examples of the hole-transporting substance included in the hole-transporting layer 5 are a triphenylamine compound such as N,N'-diphenyl-N,N'-di (m-tolyl) -benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenyl)-1,3,4-oxadiazole and 2,5-bis(1-naphthyl)-1,3,4-oxadiazole, and2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The luminescent element B shown in Figure 2 employs Alq3 as electron-transporting substance in the electron-transporting layer 6.

The thickness of each layer is the same as that of the corresponding layer in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of this invention is shown in Figure 3. This figure is an illustration showing the sectional layer structure of an example of the luminescent element, which is a multi-layer organic EL element.

The luminescent element C shown in Figure 3 comprises a substrate 1, and a transparent electrode 2, a hole-transporting layer 5, a light-emitting layer 3, an electron-transporting layer 8, and an electrode layer 4, wherein the transparent electrode and the layers are laid on substrate 1 one by one in this order.

The luminescent element C functions in the same way as the luminescent element B.

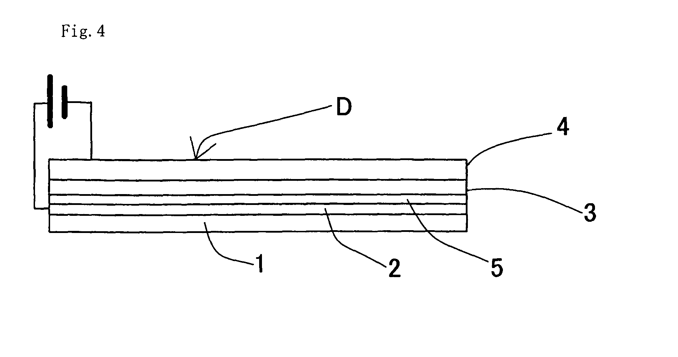

Another example of the luminescent element of this invention is shown in Figure 4. The luminescent element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer low molecular weight organic luminescent element having a hole-transporting layer that includes a hole-transporting substance and an electron-transporting light-emitting layer that includes the Nile red luminescent compound of the invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a light-emitting layer that includes a host pigment and the Nile red luminescent compound of this invention as a guest pigment, wherein the light-emitting layer is laid on the hole-transporting layer and these layers are sandwiched between the cathode and the anode. Another example is a two-layer organic luminescent element comprising a hole-transporting layer that includes a hole-transporting substance and an electron-transporting light-emitting layer that is prepared through a co-deposition of the red light-emitting compound of the invention and an electron-transporting substance, the latter layer being laid on the former, and these two layers being sandwiched between the cathode and the anode. A specific example of the second embodiment is a two-layer pigment-injected luminescent element comprising a hole-transporting layer and an electron-transporting light-emitting layer that includes a host pigment and the Nile red luminescent compound of this invention as a guest pigment, wherein the light-emitting layer is laid on the hole-transporting layer and these layers are sandwiched between the cathode and the anode. A further example is a three-layer organic luminescent element comprising a hole-transporting layer, a light-emitting layer including the Nile red luminescent compound emitting red light of this invention that is laid on the hole-transporting layer, and an electron-transporting layer that is laid on the light-emitting layer, these layers being sandwiched between the cathode and the anode.

The electron-transporting layer typically comprises 50-80% by weight of a polyvinyl carbazole (PVK), 5-40% by weight of an electron-transporting luminescent agent, and 0.01-20% by weight of the Nile red luminescent compound of the present invention. A composition within these ranges results in the emission of red light at a strong luminance.

Also, it is preferred if the light-emitting layer includes, as a sensitizing agent, rubrene, especially both of rubrene and A1q3.

A red light-emitting element utilizing the Nile red luminescent compound emitting red light of the present invention, or a white light-emitting element utilizing a blue light-emitting compound, a green light-emitting compound and the Nile red luminescent compound of the present invention may generally be used for an organic EL element driven by direct current, and also by pulses and alternating current.

The present invention will be explained in more detail by means of examples hereinafter. Needless to say, the present invention is not limited to the examples.

### Examples

### (Working Example 1)

### <Synthesis of a Brominated Nile Red Intermediate>

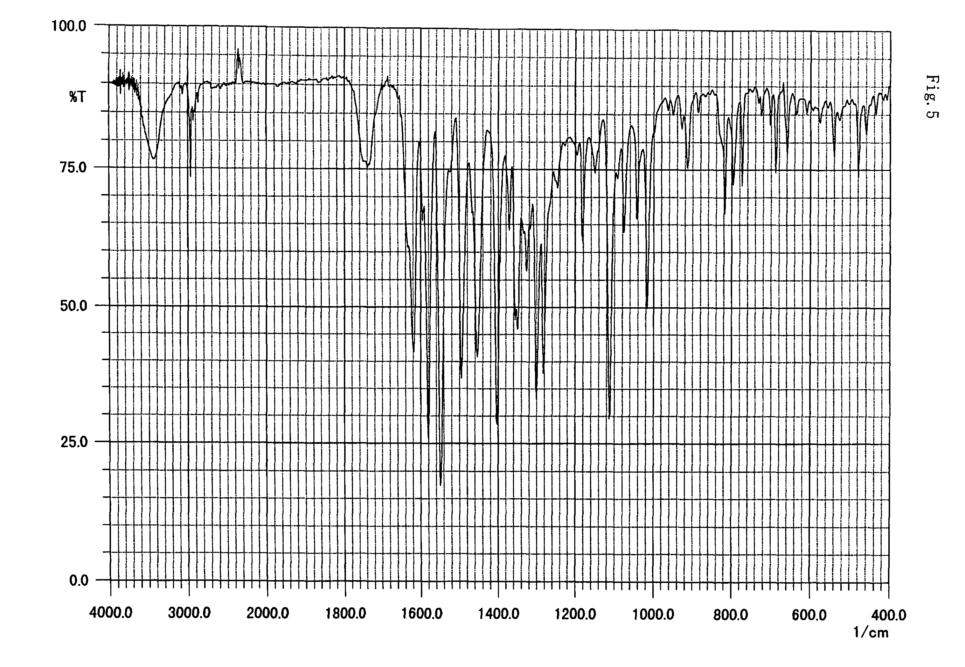

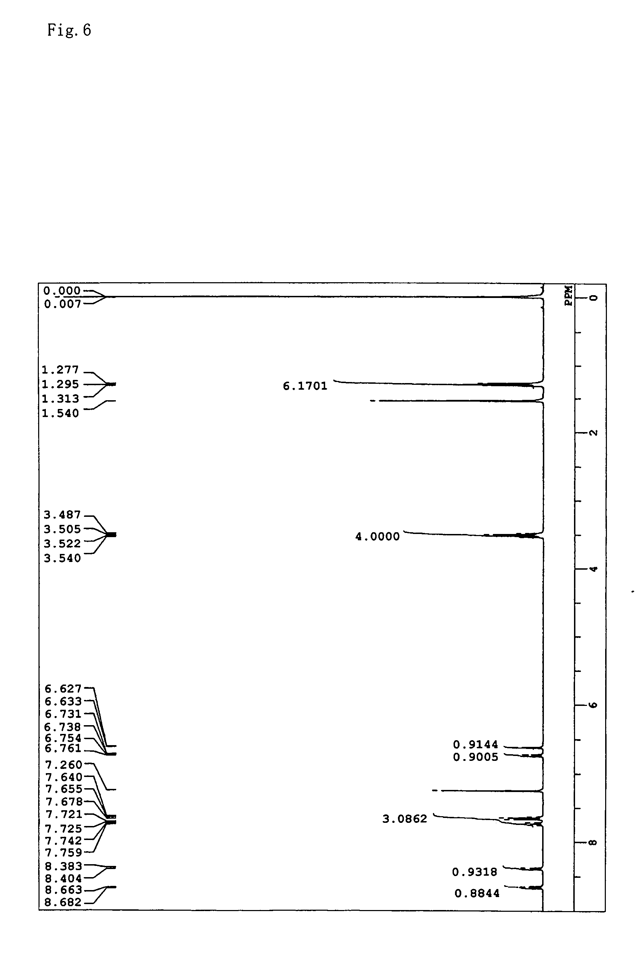

5.0g (15.7 mmol) of Nile red, 5. 63g (23.6 mmol) of diethyl bromomalonate, and 250 ml of acetic anhydride were placed in a 500 ml pear-shaped flask. The solution in the pear-shaped flask was heated in a silicone oil bath to 135°C and allowed to react for 2.5 hours at around this temperature. Acetic anhydride was distilled away with an evaporator and solids were obtained. A column, which had been filled with silica gel, was charged with the solids, and the solids were purified with benzene as a developer. 200 mg of a deep green solid matter was obtained. The yield was 3.2%. The melting point of the solid matter was 204-205°C. An IR spectrum of this deep green solid matter is shown in Figure 5, and an NMR chart of the solid matter is shown in Figure 6. The results of elemental analysis of this product are as follows.
Calculated values: C: 60.47, H: 4.31, N: 7.05, O: 8.05, Br: 20.11
Found values: C: 59.19, H: 4.24, N: 6.43, O: 8.36, Br: 21.61
Based on these results, the deep green solid matter was identified as a Nile red luminescent compound emitting red light that had the structure represented by formula (6).

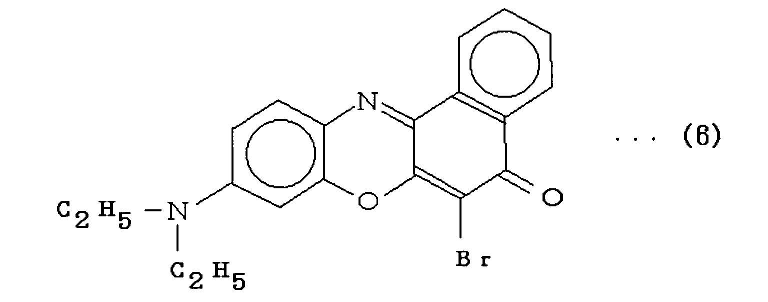

The brominated Nile red intermediate can also be synthesized by reacting Nile red with N-bromosuccinimide. Generally, N-bromosuccinimide is known as a good brominating agent that is able to replace a hydrogen atom of allyl with a high yield.

In the followings, an example of producing the brominated Nile red intermediate represented by formula (6) by using N-bromosuccinimide will be shown.

10.0 g (31.4 mmol) of Nile red, 6.20 g (34.8 mmol) of N-bromosuccinimide, 0.10 g of AIBN, and 780 ml of carbon tetrachloride were placed in a 2000 ml flask. The solution in the flask was heated in a silicone oil bath to 100°C and allowed to react for 2 hours at around this temperature. Carbon tetrachloride was distilled away with an evaporator, and solids were obtained. The solids were purified by a column chromatography that used chloroform for the developer. The purified was further recrystallized in toluene. 5.1 g of a deep green solid matter was obtained. The yield was 41%. The meltingpoint of the solid matter was 204-205°C. The employment of N-bromosuccinimide made it possible to produce the Nile red intermediate with that high yield.

### <Introduction of Trifluoromethyl Group>

In a 50 ml autoclave made of stainless steel, with a stirrer, a thermometer and a heating bath were placed 1.0 g (16.0 mmol) of copper powder that had been prepared from copper sulfate, 10 ml of dimethylformamide (DMF), which had been dried so that it contained not more than 3.0% by weight of water, and 1.98 g (5.0 mmol) of the brominated Nile red intermediate represented by formula (6) in a nitrogen atmosphere. Then the autoclave was cooled to -35°C, and 1.37 g (7.0 mmol) of trifluoromethyl iodide was also introduced into the autoclave. The mixture in the autoclave was stirred at a heated temperature of 130 to 140°C for 20 hours. Upon the termination of the reaction, the reaction product was cooled to room temperature. 20 ml of water and 15 ml of toluene were added to the cooled product, and the obtained mixture was filtered so that insoluble matters such as copper bromide were removed. Then, the organic phase was separated from the filtrate, and the separated organic liquid was washed with 30 ml of water. The washed was dried with anhydrous sodium sulfate, the dried was filtered, and the separated filtrate was concentrated with an evaporator. The obtained crude product was purified by a column chromatography that used chloroform for the developer. 1.39 g of a trifluoromethyl group-introduced product was obtained. The yield was 72%.

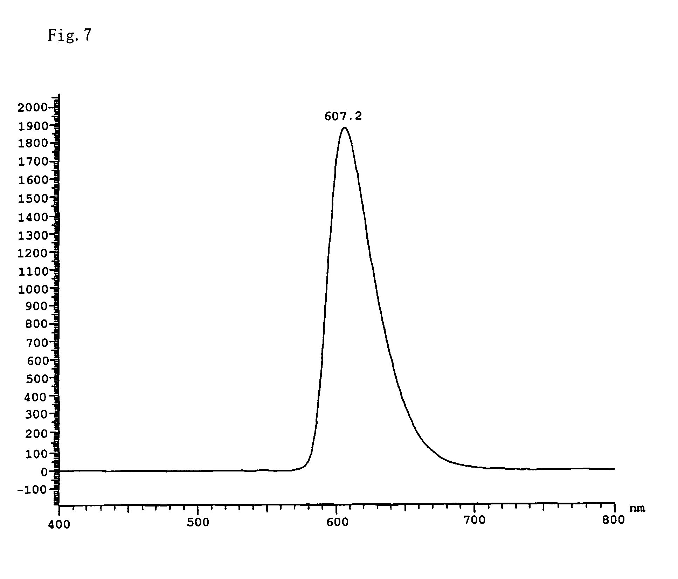

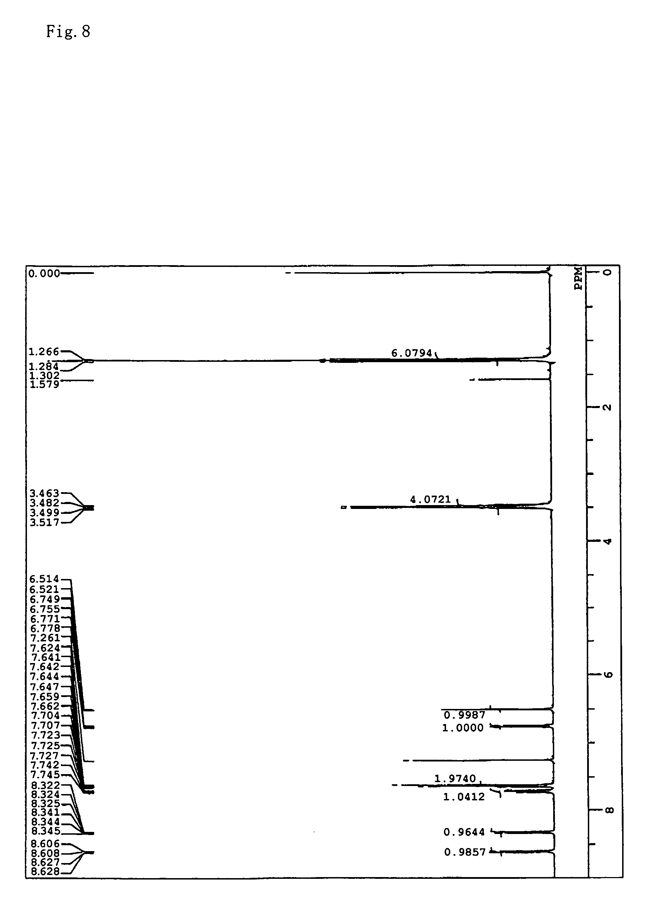

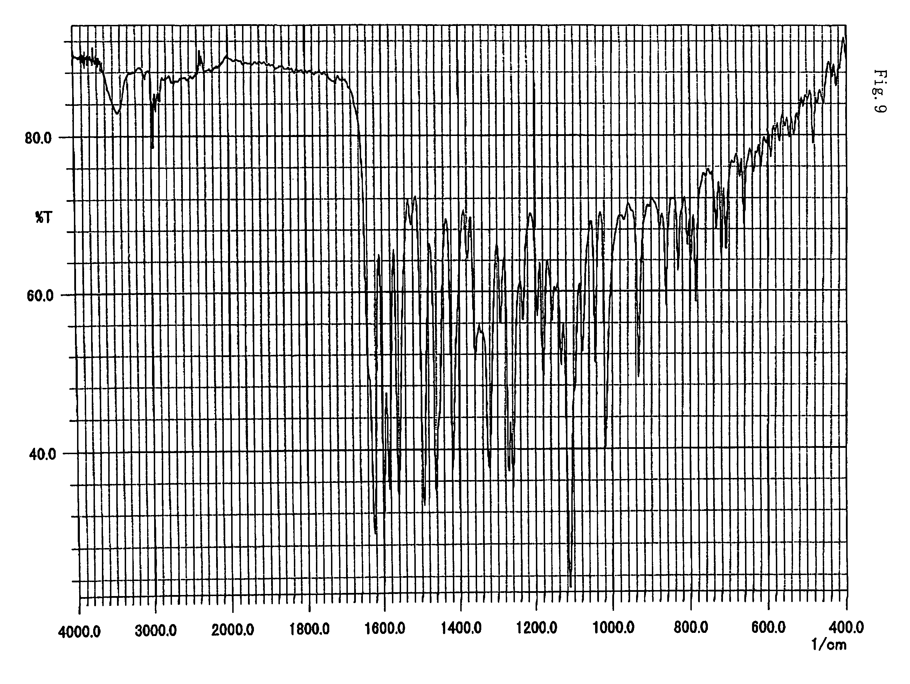

The obtained product, which was a solid matter, was purified by sublimation with a TRS-1SS apparatus produced by ULVAC-RIKO, Inc. (temperature of the high-temperature part: 190°C, temperature of the low-temperature part: 125°C, pressure: 0.5 Pa). Deep green crystals were obtained. The melting point of the crystals was from 234 to 236°C. The fluorescence spectrum of this product was measured with a model F-4500 spectrofluorophotometer (exciting wavelength: 365 nm, solvent: dioxane, concentration: 0.05% by weight). The wavelength of the maximum emission was 607.2 nm. The measured spectrum is shown in Figure 7. An NMR chart and an IR chart of the obtained product are shown in Figures 8 and 9 respectively.

The results of elemental analysis of these deep green crystals are as follows.
Calculated values: C: 65.28, H: 4.43, N: 7.25, O: 8.28, F: 14.75
Found values: C: 64.98, H: 4.36, N: 7.35, O: 8.33, F: 14.80

Based on these results, the deep green crystals were identified as a Nile red luminescent compound emitting red light that had the structure represented by formula (7).

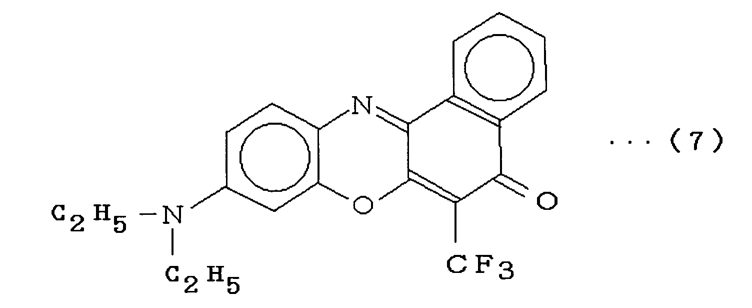

As understood from Figure 7, the fluorescence of the Nile red luminescent compound obtained in this example covers a light range the wavelengths of which are from 600 nm to 700 nm.

### (Working Example 2)

### <Introduction of Cyano Group>

In a 50 ml pear-shaped flask with a condenser were placed 0.27 g (3.0 mmol) of copper cyanide, 10 ml of dimethylformamide (DMF), which had been dried so that it contained not more than 3.0% by weight of water, and 1 g (2.52 mmol) of the brominated Nile red intermediate represented by formula (6). The mixture in the pear-shaped flask was refluxed with stirring for 4 hours. After the termination of the reaction was confirmed by thin-layer chromatography, the reaction product liquid was poured into a mixture of 10 ml of water and 3 ml of ethylenediamine. This mixture including the reaction product was extracted twice, each time with 10 ml of dichloromethane. Then, the obtained organic liquid was washed twice, each time with 10 ml of a saturated solution of salt. The washed was dried with anhydrous sodium sulfate, and the dried was filtered, so that the filtrate was separated. The solvent of the filtrate was distilled away under reduced pressure with an evaporator. The concentrated crude product was purified by a column chromatography that used silica gel for the filler. 0.76 g of crude crystals of a cyanide product was obtained. The yield was 88%.

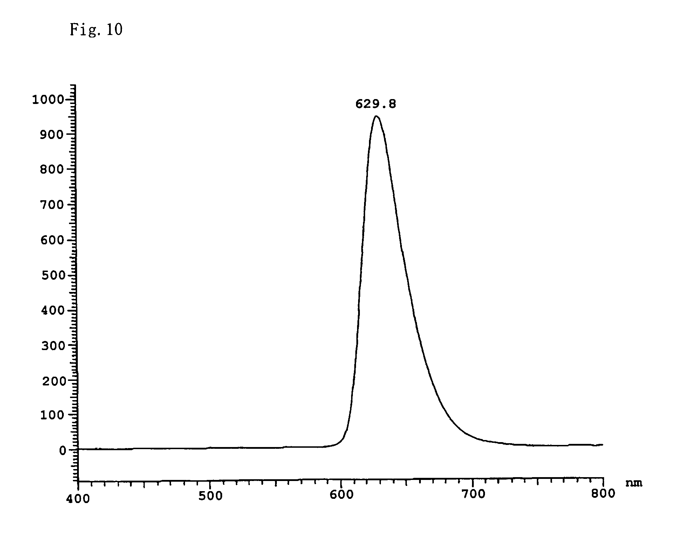

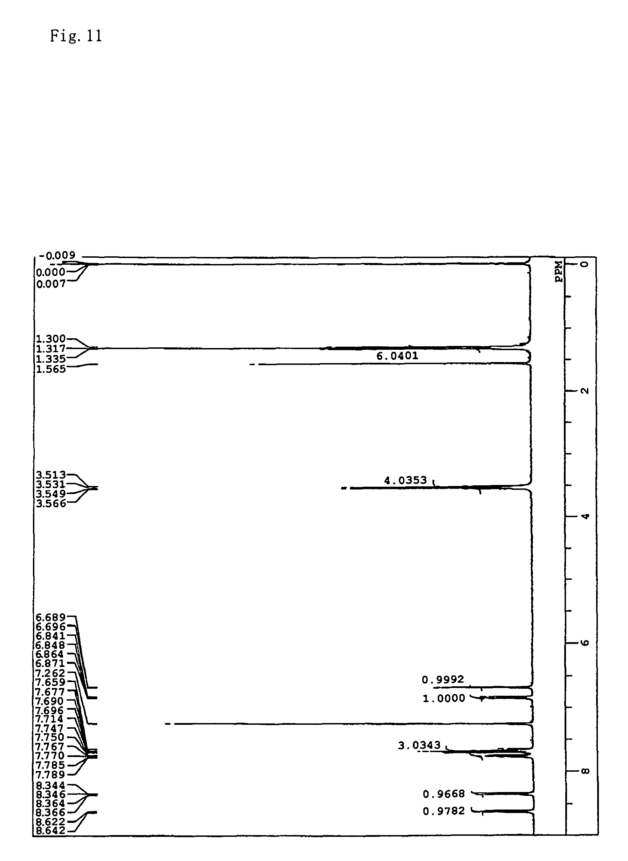

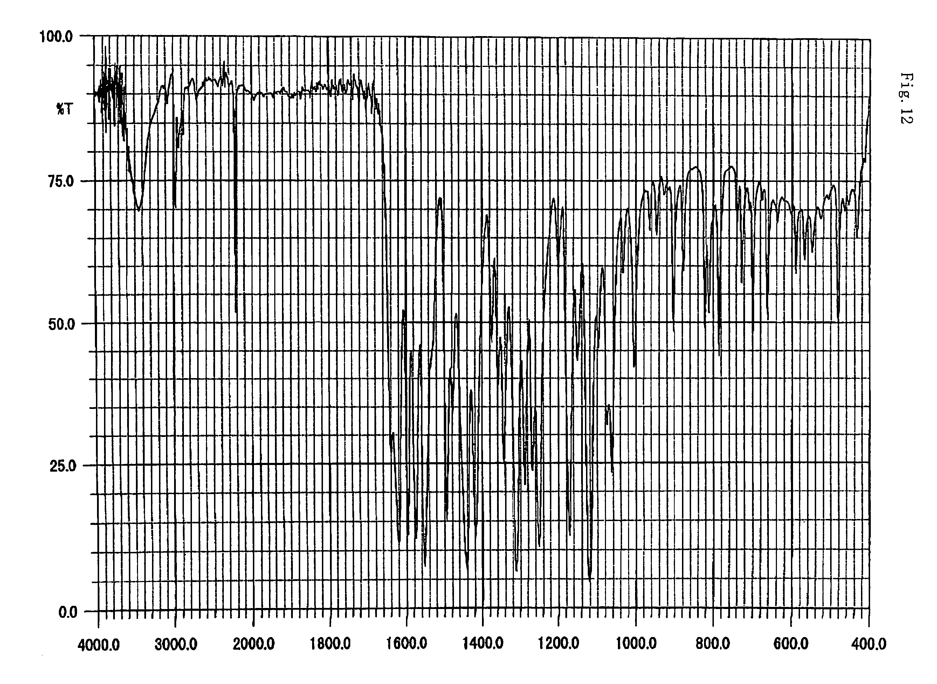

The obtained crude crystals were purified by sublimation with a TRS-1SS apparatus produced by ULVAC-RIKO, Inc. (temperature of the high-temperature part: 250°C, temperature of the low-temperature part: 150°C, pressure: 0.5 Pa). Deep green crystals were obtained. The melting point of the crystals was from 263 to 265°C. The fluorescence spectrumof this product was measured with a model F-4500 spectrofluorophotometer (exciting wavelength: 365 nm, solvent: dioxane, concentration: 0.05% by weight). The wavelength of the maximum emission was 629.8 nm. The measured spectrum is shown in Figure 10. An NMR chart and an IR chart of the obtained product are shown in Figures 11 and 12 respectively.

The results of elemental analysis of the deep green crystals are as follows.
Calculated values: C: 73.45, H: 4.99, N: 12.24, O: 9.32
Found values: C: 73.22, H: 4.95, N: 12.31, O: 9.44

Based on these results, the deep green crystals were identified as a Nile red luminescent compound emitting red light that has the structure represented by formula (8).

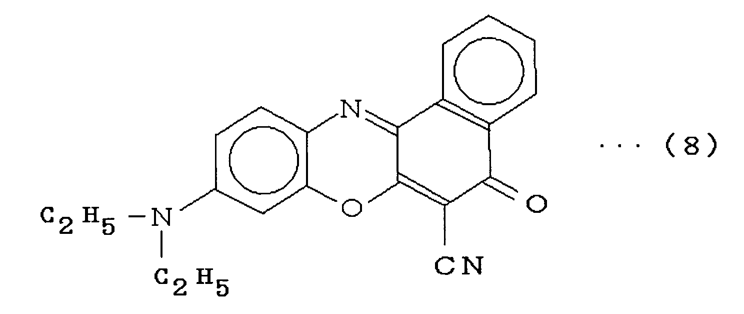

### Industrial Applicability

This invention can provide a novel Nile red luminescent compound capable of emitting at a high luminance a light that has a peak wavelength the color of which is very closer to crimson, and of enduring heat and light. Conventional technologies could not realize such luminescent compounds.

This invention can also provide a novel Nile red luminescent compound, from which a luminescent element emitting white light can be prepared.

Furthermore, this invention can provide an industrial process for easily producing the Nile red luminescent compound.

Still further, this invention can provide an EL element, the light-emitting layer of which contains the novel Nile red luminescent compound, capable of emitting a crimson light at a high luminance. Also, when the light-emitting layer includes a green light-emitting compound and a blue light-emitting compound together with the Nile red luminescent compound, a luminescent element emitting white light can be provided.

## Claims

1. A Nile red luminescent compound emitting red light that has a structure represented by formula (1): 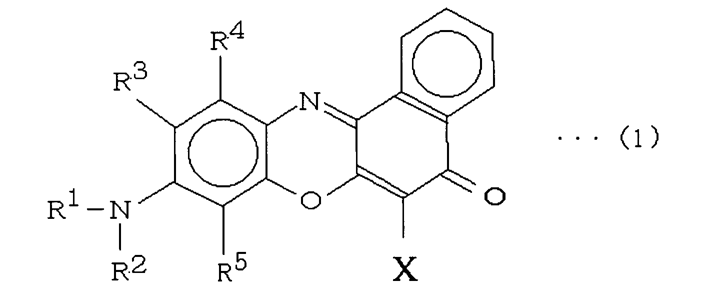 wherein R¹ is a lower alkyl group having from 1 to 5 carbon atoms or benzyl group, or forms -CH₂CH₂-CR⁶R⁷- together with R³ (wherein the carbon atom of the -CR⁶R⁷- part is bound to the benzene fragment of chemical formula (1), each of R⁶ and R⁷ is a hydrogen atom, a lower alkyl group having from 1 to 5 carbon atoms, or benzyl group, and R⁶ and R⁷ may be the same or different from each other);
R² is a lower alkyl group having from 1 to 5 carbon atoms or benzyl group, or forms -CH₂CH₂-CR⁸R⁹- together with R⁵ (wherein the carbon atom of the -CR⁸R⁹- part is bound to the benzene fragment of chemical formula (1), each of R⁸ and R⁹ is a hydrogen atom, a lower alkyl group having from 1 to 5 carbon atoms, or benzyl group, and R⁸ and R⁹ may be the same or different from each other) ;
R³ is a hydrogen atom, forms -CH₂CH₂-CR⁶R⁷- with R¹, or forms with R⁹ a naphthalene ring including as a part thereof the benzene fragment of chemical formula (1);
R⁴ is a hydrogen atom, or forms with R³ a naphthalene ring including as a part thereof the benzene fragment of chemical formula (1);
R⁵ is a hydrogen atom, or forms -CH₂CH₂-CR⁸R⁹- with R²; and
X is cyano group or a fluorohydrocarbyl group.

2. A process of producing the Nile red luminescent compound emitting red light represented by the formula (1), comprising reacting a Nile red pigment compound represented by formula (2) with a halogenating agent to produce a halogenated Nile red intermediate represented by formula (3), and replacing the halogen atom with a fluorohydrocarbyl group or cyano group: 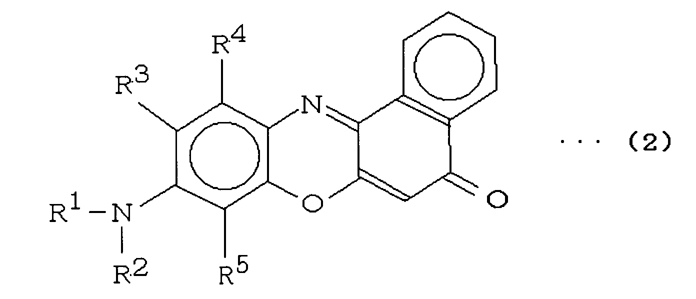 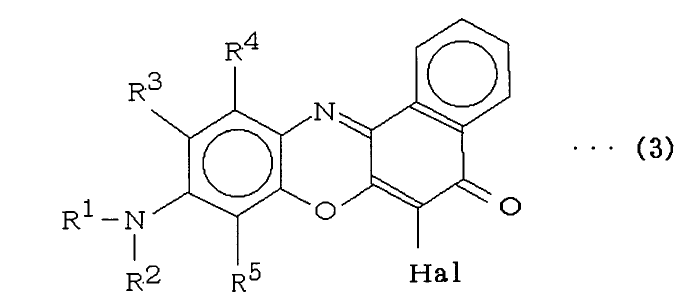 wherein R¹, R², R³, R⁴ and R⁵ mean the same atoms and groups as those defined in claim 1, and "Hal" denotes a halogen atom.

3. A luminescent element comprising a pair of electrodes and a light-emitting layer including the Nile red luminescent compound represented by formula (1) between the electrodes.
